Europäisches Patentamt

European Patent Office

Office européen des brevets

(19)

(11) Publication number: **0 172 987 B1**

## EUROPEAN PATENT SPECIFICATION

(12)

(45) Date of publication of patent specification: **17.07.91**    (51) Int. Cl.⁵: **A61K 35/30**

(21) Application number: **85104011.3**

(22) Date of filing: **03.04.85**

---

(54) Nerve regeneration.

---

(30) Priority: **04.04.84 IL 71440**

(43) Date of publication of application:
**05.03.86 Bulletin 86/10**

(45) Publication of the grant of the patent:
**17.07.91 Bulletin 91/29**

(84) Designated Contracting States:
**AT BE CH DE FR GB IT LI LU NL SE**

(56) References cited:

CHEMICAL ABSTRACTS, vol. 94, no. 11, page
30, column 1, abstract no. 76616s, 16th
March 1981, Columbus, Ohio, US; G.GROSSE:
"Experimental influence of pharmacological
agents on the regeneration of nervous tis-
sue in vitro"

CHEMICAL ABSTRACTS, vol. 97, no. 3, page
195, column 2, abstract no.18132g, 19th July
1982, Columbus, Ohio, US: M.SCHWARTZ
"Target-derived trophic factors for the adult
goldfish regenerating retina"

(73) Proprietor: **YEDA RESEARCH AND DEVELOP-
MENT COMPANY, LTD.
Weizmann Institute of Science Herzl Street
at Yavne Road P.O. Box 95
Rehovot 76100(IL)**

(72) Inventor: **Schwartz, Michal
10 Bustani Street
Rehovot(IL)**
Inventor: **Belkin, Michael
4 Alonim Street
Givat Shmuel(IL)**

(74) Representative: **Vossius & Partner
Siebertstrasse 4 P.O. Box 86 07 67
W-8000 München 86(DE)**

EP 0 172 987 B1

CHEMICAL ABSTRACTS, vol. 96, no. 7, page 351, column 1, abstract no. 65970t, 15th February 1982, Columbuso, Ohio, US; M,SCHWARTZ et al.: "Goldfish brain extract induces aggregation of acetylcholine receptors on cultured myotubes; increased activity following optic nerve axotomy"

CHEMICAL ABSTRACTS, vol. 102, no. 9, pagre 400, abstract no. 76564g, 4th March 1985, Columbus, Ohio, US; M.HADANI et al.: Substances originating from the optic nerve of neonatal rabbit induce regeneration-associated response in the injured optic nerve of adult rabbit"

## Description

The invention relates to compositions for use in the induction of regeneration of mammalian CNS.

Central nervous system (CNS) injuries comprise a considerable proportion of traumatic casualties and are the most common cause of severe permanent disability. These injuries, which include brain, spinal cord and major sense organ trauma, cause permanent disability because the adult mammalian central nervous system is incapable of functional regeneration. This is the reason why an injury in the lower spinal cord often results in the permanent confinement of the casualty to a wheel chair, why laceration in the upper spinal cord leads to paralysis of the four limbs and why a cut in the optic nerve leads to complete incurable blindness. This is also the main reason why neural transplantation operations which are technically feasible cannot be performed. Furthermore, central nervous system diseases often lead to permanent disability since the areas which were functionally affected do not regenerate. In order to be able to regenerate, the synthetic machinery in the adult mammalian neural cells has to revert to embryonal/growth synthetic pattern. Such a post-traumatic conversion takes place in the central nervous system of lower vertebrates such as fish, which are capable of full regeneration.

C.A., Vol. 94, 76616s describes the influence of pharmacological agents on the regeneration of nervous tissue in vitro.

C.A., Vol. 97, 18132g discloses the induction and support of neuritic outgrowth from the regenerating retina by an extract from adult goldfish brain.

C.A., Vol 96, 65970t describes the induction of aggregation of acetylcholine receptors on cultured myotubes by goldfish brain extract.

The present invention provides compositions for inducing a regenerative process of damages to mammalian CNS neurons. It has been found that it is possible to induce the early events of regeneration of CNS nerve cells upon contact of same with a modified environment such as that created by regenerating or neonatal CNS of lower vertebrate species or of neonatal CNS of mammals. It is also possible to induce such regeneration by a certain factor which is released from regenerating CNS of lower vertebrate species or neonatal nerves and which can be applied as such, or in concentrated form to the environment of the neuron which is to be regenerated.

According to one embodiment of the invention, the environment of the neuron to be regenerated is modified by transplanting to its vicinity a regenerating CNS nerve of a lower vertebrate species or neonatal CNS nerve of a mammal or a lower vertebrate species. The regenerating or neonatal CNS nerves secretes a substance capable of inducing regenerative response in adult CNS neurons.

Regenerative response is manifested by the following characteristics:

(1) General changes in incorporation of ($^{35}$S) methionine into retinal proteins; (2) changes in the pattern of the radiolabeled proteins; (3) ability to send out nerve fibers in culture; (4) growth and elongation of fibers, in situ.

Incorporation of ($^{35}$S) methionine revealed the results summarized in Table 1.

Both the crush and the cut injuries resulted in a general decrease in methionine incorporation by the corresponding retinal proteins, as compared to the contralateral side (L/R = 0.76±0.07).

When an optic nerve of a rabbit was cut and there was transplanted in the gap a piece of a fish or neonatal rabbit optic nerve which was transformed to a regeneration state by mechanical crushing, a few days previous to its transplantation, it was found that the procedure induced regeneration of the cut optical nerve (Table 1.).

Further experiments were carried out with material released by developing or regenerating CNS into an aqueous medium. A regenerating fish nerve or a developing mammalian nerve or brain were maintained in an aqueous medium and the medium was filtered off.

The filtrate was contacted with a cut optic nerve of the rabbit by surrounding it with a silicone tube in which this solution was provided. The results obtained were practically identical with those obtained by contact with the regenerating fish nerve.

It is evident that, when an injured rabbit optic nerve was implanted with substances originating from the regenerating fish optic nerve, a significant increase in incorporation of ($^{35}$S) methionine into the retinal proteins was observed (L/R = 1.5±0.2). Implants of silicone tubes, containing medium conditioned by injured or intact sciatic nerves, did not cause any significant effect. An irrelevant protein such as bovine serum albumin, when applied in the silicone tube to a crushed nerve, did not exhibit any effect. The incorporation was slightly increased by a medium conditioned with an intact optic nerve of fish (4R = 0.93±0.2). This may suggest the existence of trigger substances within the intact optic nerve of fish, albeit at a significantly lower level.

Substances originating from neonatal optic nerves of rabbit or brain caused a general increase in protein synthesis (L/R = 1.0±0.2) whereas media conditioned by intact nerves of adult rabbits caused only a moderate increase (L/R = 1.2±0.2). Unlike the fish where substances originating from injured optic nerve (naturally regenerating nerves) caused an increased protein synthesis, substances originating from injured rabbit optic nerve (naturally nonregenerating) were unable to cause such an increase. Similar results were obtained by using conditioned media obtained from rabbit neonatal brains

It has been found that regenerating CNS of lower vertebrate species or developing CNS of mammals secrete a substance into an aqueous medium which is highly active in inducing nerve regeneration. Such substance can be applied to the injured CNS and it stimulates CNS regeneration. The purification of the factor was effected by means of gel-filtration and reverse phase high performance liquid chromatography. Thus there was obtained a purified factor having a molecular weight in excess of about 1,000 and below about 10,000.

It is thus clear that adult mammalian CNS neurons can be activated by contact with appropiate factors present in regenerating lower vertebrates CNS or in neonatal mammalian CNS.

These phenomena have not been previously detected in the CNS of adult mammals following injury.

Gel analysis of the radiolabeled retinal proteins show that increased labeling, induced by substances originating from the regenerating fish optic nerve, was manifested selectively in a few polypeptides. These can be considered as putative growth associated proteins having the following molecular weights: 130K, 110K, 74K, 64K, 26K.

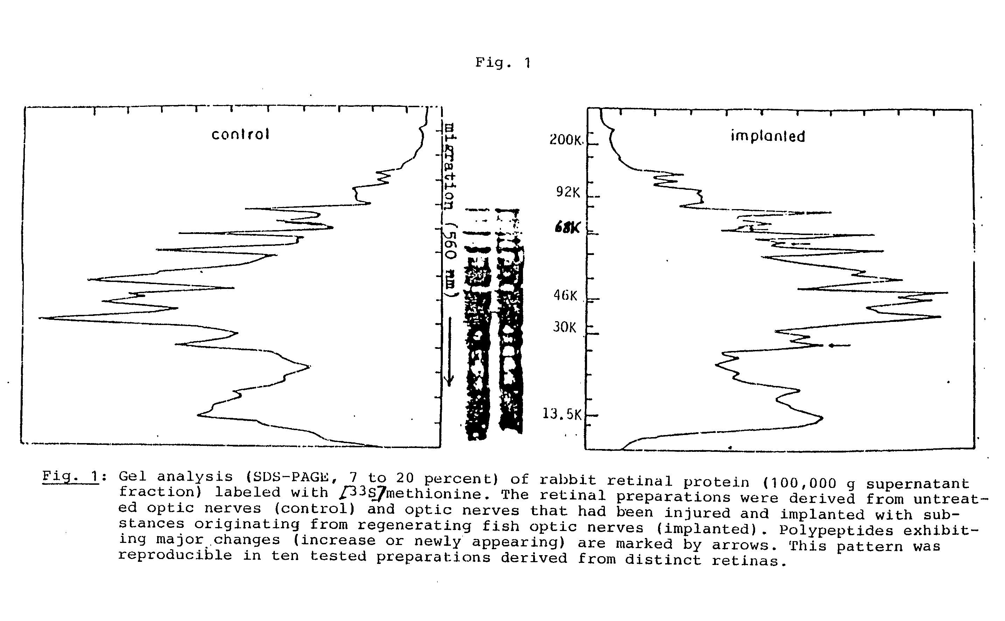

The retinal proteins in the contralateral side were used as a control . Under the same experimental conditions, proteins of a retina of an injured nerve that had not been implanted, displayed a pattern identical to that of the uninjured side. The altered protein pattern of the rabbit retina which was induced by the grafts of the regenerating fish optic nerve (introduced into the injured rabbit optic nerve) resembles that observed in regenerating CNS of lower vertebrates and mammalian CNS (Fig.1).

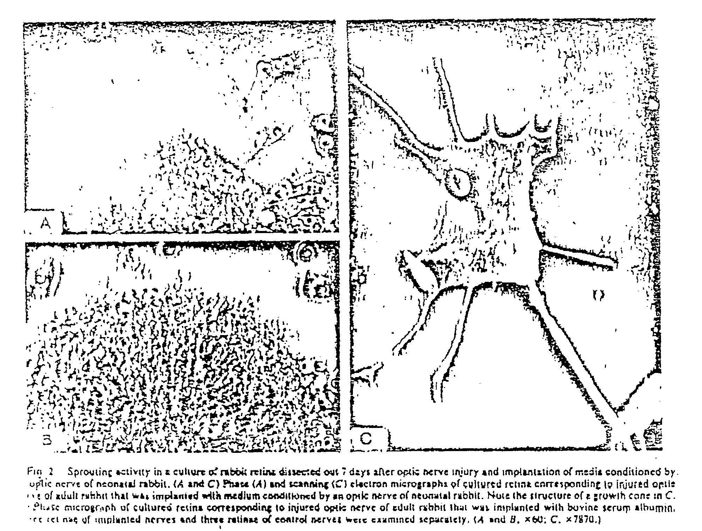

In order to determine if the induced biochemical changes are associated with regeneration activity, we measured the ability of the retina to sprout in culture. Adult mammalian CNS neurons of intact or injured nerves do not send out neurites in culture. However,lower vertebrates CNS neurons express high sprouting ability in culture following nerve injury. Retinae of injured rabbit optic nerves, into which segments of regenerating fish optic nerve were transplanted, did display sprouting activity in culture. To verify the nature of the fibers emerging from the retinae of the transplanted nerve, we used a scanning electron microscope which showed the typical structure of a growth cone at the growing tip (Fig. 2).

Preliminary results have also pointed out the effect of the implanted substances on growth, in situ, as was revealed by electron microscopical analysis.

Light microscopical analysis using horse-radish peroxidase for retrograde labeling of the retina revealed the higher frequency of new sprouts and surviving ganglion cells in retina of injured and implanted nerve. This effect was so far noticed up to one month after the surgical manipulation.

The study demonstrates that the visual system of an adult rabbit (an example of mammalian CNS) can express regeneration characteristics if appropriate external signals are provided. Such signals are supplied in this work by diffusible substances that originate from non-neuronal cells of regenerating fish optic nerve, mammalian neonatal optic nerves and brains.

It appears that the state of the non-neuronal cells which provide the signals is crucial. Thus, conditioned medium of regenerating fish optic nerve was effective whereas media conditioned by an intact fish optic nerve has a small effect. Furthermore, these signals seem to have a certain degree of specificity. Thus, a xenogeneic regenerating CNS was able to produce such signals, while intact or injured nerves of syngenic peripheral nerve failed to trigger regeneration response. These observations imply that adult rabbit retinal ganglion cells can be induced to regenerate by diffusible external signals produced by glial cells of regenerating systems rather than Schwann cells. Furthermore, the results indicate that the regeneration response machinery within the adult rabbit retina, responsible for receiving the triggering signals, is intact and evolutionary conserved. It therefore seems that the microenvironment of the adult rabbit optic nerve, unlike that of the fish is either insufficient by itself, or defective with respect to generation of signals necessary for triggering cell body response.

EXPERIMENTAL PROCEDURE :

The optic nerves of the anesthesized rabbits were exposed distally from the globe for about 1 cm. Unilateral (left side) crush or cut injuries were then performed at a distance of 4-5 mm from the optic disc.

When a regenerating fish optic nerve, (5 to 6 days after crush injury of the fish nerve) was transplanted, it was sutured by 3 absorbable sutures through the respective meninges. When implantation was performed the rabbit optic nerve was crushed and a sleeve of a silicone tube (2 mm internal diameter) was placed around the injured site. The silicone tube was coated prior to the implantation with collagen, onto which medium conditioned by regenerating fish nerves was applied. This conditioned medium was prepared by incubating segments of a fish nerve in a serum-free medium (2 segments/250$\mu$l, DMEM, Gibco) for 1.5 h at room temperature.

The invention is further illustrated with reference to the enclosed Figures, in which:

Fig. 1     illustrates gel analysis results of rabbit retinal protein;

Fig. 2     Illustrates the sprouting activity in a culture of rabbit retina after optic nerve injury.

Table 1.     Increased [$^{35}$S]methionine incorporation by rabbit retinal proteins induced by diffusible substances grafted into injured adult rabbit optic nerves.

| Group | L/R $\bar{X} \pm SEM$ |
|---|---|
| Crush only | 0.76±0.07 |
| Transplanted (Regenerating fish optic nerve) | 1.8 ±0.5 |
| Implanted (CM of regenerating fish optic nerve) | 1.5 ±0.2 |
| Implanted (CM of intact fish optic nerve) | 0.93±0.2 |
| Implanted (CM of injured optic nerve of adult rabbit) | 0.8 ±0.2 |
| Implanted (CM of intact optic nerve of adult rabbit) | 1.15±0.2 |
| Implanted (CM of optic nerve of neonatal rabbit) | 1.87±0.2 |
| Implanted (CM of injured adult optic nerve implanted with CM of neonatal rabbit optic nerve) | 1.63±0.3 (SD) |

One week following the rabbit optic nerve injury and the surgical manipulations, the retinae of the various experimental groups were dissected out and pulse labeled in vitro, with [$^{35}$S]methionine. The ratio between specific activities of the labeled proteins in the left (operated) versus the right (unoperated contralateral) sides was used as a test parameter (defined as L/R).

CM = conditioned medium.

5

## Claims

1. A composition containing a central nervous system (CNS) nerve-regeneration triggering substance derived from neonatal or injured nerves of CNS of a lower vertebrate species or of neonatal nerves of mammals for use as a medicament.

2. A composition containing a central nervous system (CNS) nerve-regeneration triggering substance derived from neonatal or injured nerves of CNS of a lower vertebrate species or of neonatal nerves of mammals for inducing regeneration of nerves of the mammalian CNS.

3. A composition according to claim 1 or 2 containing an adequate quantity of the CNS-nerve regeneration triggering substance wherein the active substance is derived from an injured nerve of CNS of a lower vertebrate species, such as fish.

4. A composition aocording to claim 1 or 2 wherein the active substance is derived from neonatal nerves of CNS of fish or other lower vertebrate species.

5. A composition according to claim 1 or 2, wherein the active substance is a crushed regenerating nerve of CNS of a lower vertebrate species.

6. A composition according to claim 1 or 2, wherein the active substance is contained in a fluid obtained by filtering off a comminuted regenerating nerve of CNS of a lower vertebrate species, of embryonic nerves of CNS or of neonatal nerves of CNS.

7. A composition according to any of claims 1 to 6, wherein the active substance has a M.W. between 1,000 and 10,000.

## Claims for the following Contracting State: AT

1. Use of a central nervous system (CNS) nerve-regeneration triggering substance derived from neonatal or injured nerves of CNS of a lower vertebrate species or of neonatal nerves of mammals for the preparation of a medicament for inducing regeneration of nerves of the mammalian CNS.

2. Use according to claim 1 whereby an adequate quantity of the CNS-nerve regeneration triggering substance wherein the active substance is derived from an injured nerve of CNS of a lower vertebrate species, such as fish, is used.

3. Use according to claim 1, whereby the active substance is derived from neonatal nerves of CNS of fish or other lower vertebrate species.

4. Use according to claim 1, whereby the active substance is a crushed regenerating nerve of CNS of a lower vertebrate species.

5. Use according to claim 1, whereby the active substance is contained in a fluid obtained by filtering off a comminuted regenerating nerve of CNS of a lower vertebrate species, of embryonic nerves of CNS or of neonatal nerves of CNS.

6. Use according to any of claims 1 to 5, whereby the active substance has a M.W. between 1,000 and 10,000.

## Revendications

1. Composition comprenant une substance initialisant la regénération nerveuse du système nerveux central (SNC), ladite substance provenant de nerfs néonatals ou de nerfs endommagés du SNC d'une espèce de vertébré inférieur ou de nerfs néonatals de mammifère, pour utilisation en tant que médicament.

2. Composition comprenant une substance initialisant la regénération nerveuse du système nerveux central (SNC), ladite substance provenant de nerfs néonatals ou de nerfs endommagés du SNC d'une espèce de vertébré inférieur ou de nerfs néonatals de mammifère, pour induire la regénération de nerfs du SNC de mammifère.

3. Composition suivant la revendication 1 ou 2, contenant une quantité appropriée de ladite substance initialisant la regénération nerveuse du SNC, dans laquelle la substance active provient d'un nerf endommagé du SNC d'une espèce de vertébré inférieur, notamment de poisson.

4. Composition suivant la revendication 1 ou 2, dans laquelle la substance active provient de nerfs néonatals du SNC de poisson ou d'une autre espèce de vertébré inférieur.

5. Composition suivant la revendication 1 ou 2, dans laquelle la substance active est un nerf regénérant broyé du SNC d'une espèce de vertébré inférieur.

6. Composition suivant la revendication 1 ou 2, dans laquelle la substance active est contenue dans un fluide obtenu par filtration d'un nerf regénérant réduit en fragments du SNC d'une espèce de vertébré inférieur, de nerfs embryonnaires du SNC ou de nerfs néonatals du SNC.

7. Composition suivant l'une quelconque des revendications 1 à 6, dans laquelle la substance active a un poids moléculaire compris entre 1 000 et 10 000.

**Revendications pour l'Etat contractant suivant: AT**

1. Utilisation d'une substance initialisant la regénération nerveuse du système nerveux central (SNC), ladite substance provenant de nerfs néonatals ou de nerfs endommagés du SNC d'une espèce de vertébré inférieur ou de nerfs néonatals de mammifère, pour induire la regénération de nerfs du SNC de mammifère.

2. Utilisation suivant la revendication 1, selon laquelle on utilise une quantité appropriée de ladite substance initialisant la regénération nerveuse du SNC, dans laquelle la substance active provient d'un nerf endommagé du SNC d'une espèce de vertébré inférieur, notamment de poisson.

3. Utilisation suivant la revendication 1, dans laquelle la substance active provient de nerfs néonatals du SNC de poisson ou d'une autre espèce de vertébré inférieur.

4. Utilisation suivant la revendication 1, dans laquelle la substance active est un nerf regénérant broyé du SNC d'une espèce de vertébré inférieur.

5. Utilisation suivant la revendication 1, dans laquelle la substance active est contenue dans un fluide obtenu par filtration d'un nerf regénérant réduit en fragments du SNC d'une espèce de vertébré inférieur, de nerfs embryonnaires du SNC ou de nerfs néonatals du SNC.

6. Utilisation suivant l'une quelconque des revendications 1 à 5, dans laquelle la substance active a un poids moléculaire compris entre 1 000 et 10 000.

**Patentansprüche**

1. Mittel, enthaltend eine Substanz, die die Nervenregenerierung des zentralen Nervensystems (ZNS) auslöst, welche von Neugeborenen- oder verletzten Nerven des ZNS einer niederen Wirbeltierart oder von Neugeborenen-Nerven von Säugern stammt, zur Verwendung als Medikament.

2. Mittel, enthaltend eine Substanz, die die Nervenregenerierung des zentralen Nervensystems (ZNS) auslöst, welche von Neugeborenen- oder verletzten Nerven des ZNS einer niederen Wirbeltierart oder von Neugeborenen-Nerven von Säugern stammt, zur Induktion der Regenerierung von Nerven des Säuger-ZNS.

3. Mittel nach Anspruch 1 oder 2, enthaltend eine adäquate Menge der die ZNS-Nervenregenerierung auslösenden Substanz, wobei der Wirkstoff von einem verletzten Nerv des ZNS einer niederen Wirbeltierart, wie z.B. eines Fisches, stammt.

4. Mittel nach Anspruch 1 oder 2, wobei der Wirkstoff von Neugeborenen-Nerven des ZNS eines Fisches oder einer anderen niederen Wirbeltierart stammt.

5. Mittel nach Anspruch 1 oder 2, wobei der Wirkstoff ein zerguetschter regenerierender Nerv des ZNS einer niederen Wirbeltierart ist.

6. Mittel nach Anspruch 1 oder 2, wobei der Wirkstoff in einer Flüssigkeit enthalten ist, die durch Abfiltern eines zerkleinerten regenerierenden Nerves des ZNS einer niederen Wirbeltierart, von embryonalen Nerven des ZNS oder von Neugeborenen-Nerven des ZNS erhalten wird.

7. Mittel nach einem der Ansprüche 1 bis 6, wobei der Wirkstoff ein Molekulargewicht zwischen 1 000 und 10 000 aufweist.

**Patentansprüche für folgenden Vertragsstaat: AT**

1. Verwendung einer Substanz, die die Nervenregenerierung des zentralen Nervensystems (ZNS) auslöst, welche von Neugeborenen- oder verletzten Nerven des ZNS einer niederen Wirbeltierart oder von Neugeborenen-Nerven von Säugern stammt, für die Herstellung eines Medikaments zur Induzierung der Regenerierung von Nerven des Säuger-ZNS.

2. Verwendung nach Anspruch 1, wobei eine adäquate Menge einer die ZNS-Nervenregenerierung auslösenden Substanz verwendet wird, wobei der Wirkstoff von einem verletzten Nerv des ZNS einer niederen Wirbeltierart, wie z.B. eines Fisches, stammt.

3. Verwendung nach Anspruch 1, wobei der Wirkstoff von Neugeborenen-Nerven des ZNS von Fischen oder anderen niederen Wirbeltierarten stammt.

4. Verwendung nach Anspruch 1, wobei der Wirkstoff ein zerquetschter regenerierender Nerv des ZNS einer niederen Wirbeltierart ist.

5. Verwendung nach Anspruch 1, wobei der Wirkstoff in einer Flüssigkeit enthalten ist, die durch Abfiltern eines zerkleinerten regenerierenden Nerves des ZNS einer niederen Wirbeltierart, von embryonalen Nerven des ZNS oder von Neugeborenen-Nerven des ZNS erhalten wird.

6. Verwendung nach einem der Ansprüche 1 bis 5, wobei der Wirkstoff ein Molekulargewicht zwischen 1 000 und 10 000 aufweist.

Fig. 1

**Fig. 1:** Gel analysis (SDS-PAGE, 7 to 20 percent) of rabbit retinal protein (100,000 g supernatant fraction) labeled with $[^{33}S]$methionine. The retinal preparations were derived from untreated optic nerves (control) and optic nerves that had been injured and implanted with substances originating from regenerating fish optic nerves (implanted). Polypeptides exhibiting major changes (increase or newly appearing) are marked by arrows. This pattern was reproducible in ten tested preparations derived from distinct retinas.

EP 0 172 987 B1

Fig. 2  Sprouting activity in a culture of rabbit retina dissected out 7 days after optic nerve injury and implantation of media conditioned by optic nerve of neonatal rabbit. (A and C) Phase (A) and scanning (C) electron micrographs of cultured retina corresponding to injured optic nerve of adult rabbit that was implanted with medium conditioned by an optic nerve of neonatal rabbit. Note the structure of a growth cone in C. Phase micrograph of cultured retina corresponding to injured optic nerve of adult rabbit that was implanted with bovine serum albumin. ... retinae of implanted nerves and three retinae of control nerves were examined separately. (A and B, ×60; C. ×7870.)